# EUROPEAN PATENT APPLICATION

(11) **EP 2 030 964 A1**
(43) Date of publication of application: **04.03.2009**
(21) Application number: 08013632.8
(22) Date of filing: 29.07.2008
(51) Int. Cl.: C07C 201/04, C07C 203/00

(54) **Process for producing esters of nitrous acid**

(30) Priority: 06.08.2007 JP 2007204025; 18.07.2008 JP 2008187101
(71) Applicant: Daicel Chemical Industries, Ltd., Kita-ku, Osaka-shi Osaka 530-0001 (JP)
(72) Inventor: Shibamoto, Akihiro, Himeji-shi Hyogo 671-1283 (JP); Iwahama, Takahiro, Himeji-shi Hyogo 671-1283 (JP)
(74) Representative: Grünecker, Kinkeldey, Stockmair & Schwanhäusser Anwaltssozietät

(57) **Abstract**

A process for producing an esther of nitrous acid includes allowing a nitrogen oxide to react with an alcohol at a reaction temperature lower than 10°C.

## Description

### FIELD OF THE INVENTION

The present invention relates to a process for producing a nitrite (or an ester of nitrous acid) from an alcohol and a nitrogen oxide.

### BACKGROUND OF THE INVENTION

A nitrite (for example, an alkyl nitrite) is used as an additive for automotive oil, a stabilizer for an unsaturated organic compound, a pharmaceutical preparation, a reagent (for example, a reagent for oximation, nitrosation, or diazotization), and an intermediate for chemical synthesis.

So far, various processes for producing such a nitrite have been reported. For example, Japanese Patent Application Laid-Open No. 529576/2003 (JP-2003-529576A, Claims and Examples) and Synthesis 2005, Vol.11, p.1803 disclose a process for allowing an alcohol (such as n-butanol, t-butanol, or isoamyl alcohol) to react with a salt of nitrous acid (e.g. , sodium nitrite). However, this process is not industrially preferable because of the formation of stoichiometric quantities of a metal salt as a by-product.

On the other hand, Japanese Patent Application Laid-Open No. 298706/1994 (JP-6-298706A, Claims and Examples) discloses a process for producing a C₁-C₄alkyl nitrite by a reaction of a C₁-C₄alkanol, a nitrogen oxide, and oxygen. In this process, the reaction is conducted by feeding the oxygen and the nitrogen oxide from the bottom of a reactor vessel designed as a gas-washing tower. The nitrogen oxide is a mixture at least containing nitrogen monoxide in an amount of 50% by mole relative to the total gram atomic number of nitrogen atoms in the nitrogen oxide and is mixed with one or more inactive gas components which hold 0 to 90% by volume of the total amount of gas to be used. The amount of oxygen is 0.15 to 0.3 mole per mole of NO. The number of moles of NO is larger than that of NO₂. The amount of the alkanol is 0.8 to 2 mole per gram atom of nitrogen atom of the nitrogen oxide, and 5 to 60% of the total amount of the alkanol is injected in the state of steam or mist to the bottom of the reactor vessel. The remaining alkanol is fed to the upper part of the reactor vessel. The reaction temperature and the reaction pressure are 10 to 150°C and 0.5 to 6 bar, respectively, and the residence time of the reaction components in the reactor vessel is set up as 1 to 500 seconds. Incidentally, the reason for that the reaction temperature is 10 to 150°C is probably that the process described in this document requires the use of the nitrogen oxide in a gaseous state.

This document mentions that the C₁-C₄alkanol includes, for example, methanol, ethanol, propanol, isopropanol, butanol, sec-butanol, and i-butanol, preferably methanol and ethanol, particularly preferably methanol, and that the resulting product is preferably methyl nitrite and ethyl nitrite, particularly preferably methyl nitrite. In all examples of the document, methyl nitrite is produced.

The process described in the document is free from the above-mentioned formation of metal salts as by-products. In addition, the process is industrially preferable since the nitrogen oxide is inexpensive. However, the process described in the document tends to lower the reaction yield relatively. In particular, nitrous acid esters of secondary and tertiary alcohols tend to be hydrolyzed and are unstable, which further lowers the yields of these esters. That is, according to the process described in the document, it is difficult to produce a nitrous acid ester of a secondary or tertiary alcohol efficiently.

Therefore, a process for efficiently producing a nitrite from the nitrogen oxide, which is inexpensive, has been required.

### SUMMARY OF THE INVENTION

It is therefore an object of the present invention to provide an efficient process for producing a nitrite by using a nitrogen oxide.

Another object of the present invention is to provide an efficient process for producing a nitrous acid ester of a secondary or tertiary alcohol in a high yield.

The inventors of the present invention made intensive studies to achieve the above objects and finally found that a reaction (esterification) of an alcohol and a nitrogen oxide under a specific low temperature condition realizes an efficient production of a nitrite. In particular, the inventors found that the reaction realizes the production of a nitrous acid ester of a secondary or tertiary alcohol in a high yield, which the conventional process cannot produce in a sufficient yield under the above-mentioned condition. The present invention has been accomplished based on the above findings.

That is, the process of the present invention comprises allowing a nitrogen oxide to react with an alcohol at a reaction temperature lower than 10°C (for example, a reaction temperature of -25°C to 3°C) to produce a nitrite. In the process, the nitrogen oxide may contain at least N₂O₃. In the process, a liquid nitrogen oxide may be used. For example, the reaction may be carried out by bringing the liquid N₂O₃ into contact with the alcohol or by vaporizing the liquid N₂O₃ and bringing the vaporized N₂O₃ into contact with the alcohol.

In the process of the present invention, a nitrite corresponding to the alcohol is efficiently produced. In particular, although a nitrite cannot be obtained in a sufficient yield by the conventional process comprising a reaction of a secondary or tertiary alcohol and a nitrogen oxide, the efficient production of the nitrite comprising the reaction of the secondary or tertiary alcohol and the nitrogen oxide is accomplished according to the present invention. Therefore, in the process of the invention, the alcohol may be, particularly, an aliphatic secondary alcohol or an aliphatic tertiary alcohol.

Moreover, in the process, the reaction may be performed in the presence of a nitrite corresponding to the alcohol.

### DETAILED DESCRIPTION OF THE INVENTION

In the present invention, a nitrite is produced by a reaction of a nitrogen oxide and an alcohol (including a phenol compound) at a reaction temperature lower than 10°C.

The nitrogen oxide is not particularly limited to a specific one as long as the nitrogen oxide reacts with an alcohol to generate a nitrite. The nitrogen oxide usually comprises at least one member selected from the group consisting of NO (nitrogen monoxide), NO₂ (nitrogen dioxide), N₂O₃ (dinitrogen trioxide), and N₂O₄ (dinitrogen tetroxide). In particular, it is preferable that the nitrogen oxide at least contain N₂O₃.

The nitrogen oxide to be used for the reaction (or the nitrogen oxide before contacting with the alcohol) may be either in a liquid state or in a gaseous state. In particular, the nitrogen oxide may at least contain a liquid nitrogen oxide (particularly a nitrogen oxide containing at least liquid N₂O₃).

Incidentally, the liquid nitrogen oxide may be vaporized and then subjected to the reaction (that is, the liquid nitrogen oxide may be vaporized and the vaporized nitrogen oxide may be brought into contact with the alcohol). For example, in the use of the nitrogen oxide containing liquid N₂O₃, the liquid N₂O₃ may directly be brought into contact with the alcohol, or the nitrogen oxide containing vaporized N₂O₃ may be brought into contact with the alcohol. Incidentally, the liquid N₂O₃ can be obtained, for example, by allowing NO to contact (or react) with NO₂ or oxygen at a relatively low temperature [for example, not higher than 3°C (e.g., about -30°C to 2°C, preferably about -20°C to 0°C)].

Moreover, in addition to the nitrogen oxide, oxygen may be used (or oxygen may coexist in the reaction). The coexistence with oxygen or the use of a mixture of two or more kinds of nitrogen oxides makes each nitrogen oxide being in the state of equilibrium with each other. The mixture being in the state of equilibrium may be used as the nitrogen oxide for the reaction. In particular, a mixture of NO and NO₂ or a mixture of NO and oxygen is preferably used to shift the equilibrium toward the formation of N₂O₃. Further, the nitrogen oxide (or oxygen) may be diluted with an inactive gas (such as nitrogen or argon).

The alcohol is not particularly limited to a specific one as long as the alcohol is a compound represented by the following formula:

R(OH)n

wherein R represents an organic group, and n denotes an integer of not less than 1.

Various compounds represented by the formula may be used as the alcohol.

In the above formula, n is not particularly limited to a specific number as long as the number is an integer of not less than 1. For example, the number n is 1 to 6, preferably 1 to 4, more preferably 1 to 3, and particularly 1 to 2.

Concrete examples of the alcohol include an aliphatic alcohol, an aromatic alcohol [or a phenol compound, for example, amono- to tetrahydroxyC₆₋₂₀arene such as phenol, cresol, naphthol, or hydroquinone], and others.

The aliphatic alcohol (an alcohol of an aliphatic hydrocarbon) may include a primary alcohol {for example, an alkanol (e.g., a C₁₋₂₀alkanol such as methanol, ethanol, n-propanol, n-butanol, n-pentanol, or n-hexanol, preferably a C₁₋₁₀alkanol, and more preferably a C₁₋₆alkanol), an araliphatic alcohol or a hydrogenated product thereof (for example, benzyl alcohol and phenethyl alcohol), and a polyol [for example, an alkanepolyol (e. g. , an alkanedi- to tetraol such as ethylene glycol, 1,3-propanediol, 1,4-butanediol, trimethylolethane, trimethylolpropane, or pentaerythritol), a polyalkanepolyol (e.g., diethylene glycol), a cycloalkanepolyalkanol (e.g., cyclohexanedimethanol), and xylylene glycol]}, a secondary alcohol {for example, an alkanol (for example, a C₃₋₂₀alkanol such as 2-propanol (isopropanol), 2-butanol (sec-butanol), 2-pentanol (sec-amyl alcohol), 3-pentanol, 2-hexanol, or 3-hexanol, preferably a C₃₋₁₀alkanol, and more preferably a C₃₋₆alkanol), a cycloalkanol (for example, a C₄₋₁₀cycloalkanol such as cyclohexanol, and preferably a C₅₋₈cycloalkanol), a polycycloalkanol (a bi- to tetracycloalkanol such as 2-adamantanol), and a polyol [for example, an alkanepolyol (for example, an alkanedi- to tetraol such as propylene glycol or glycerin); and a cycloalkanepolyol such as a cycloalkanediol (for example, a C₄₋₁₀cycloalkanediol such as cyclohexanediol (e.g., 1,4-cyclohexanediol), and preferably a C₅₋₈cycloalkanediol)]}, a tertiary alcohol (for example, an alkanol (for example, a C₄₋₂₀alkanol such as t-butanol or 2-methyl-2-butanol (t-amyl alcohol), preferably a C₄₋₁₀alkanol, and more preferably a C₄₋₆alkanol), a polycycloalkanol (for example, a bi- to tetracycloalkanol such as 1-adamantanol), a triphenylmethanol, and a polyol [for example, an alkanepolyol (e.g., an alkanedi- to tetraol such as pinacol)]}.

Incidentally, the alcohol also includes an alcohol having a substituent (for example, a halogen atom, a cyano group (nitryl group), a carboxyl group, an ether group, and a nitro group).

Among these alcohols, the preferred alcohol includes an aliphatic alcohol. Moreover, in the present invention, even in the use of a secondary alcohol or a tertiary alcohol, particularly, an aliphatic secondary alcohol [for example, an alkanol (a secondary alkanol, e. g. , a secondary C₃₋₁₀alkanol such as 2-butanol or 2-pentanol) and a cycloalkanediol (e.g., a C₅₋₈cycloalkanediol such as cyclohexanediol)] or an aliphatic tertiary alcohol [for example, an alkanol (for example, a tertiary alkanol, e.g. , a tertiary C₄₋₁₀alkanol such as t-butanol or t-amyl alcohol)], the corresponding nitrite can be obtained in a high yield. Therefore, these alcohols may be preferably used.

Incidentally, the alcohol may be in the state of gas, liquid, or solid at a reaction temperature (a temperature lower than 10°C) or in the reaction system.

The amount of the nitrogen oxide may be, for example, about 0.1 to 5 equivalents, preferably 0.15 to 4 equivalents, and more preferably 0.2 to 3 equivalents relative to the hydroxyl group of the alcohol.

Incidentally, the contact process of the nitrogen oxide and the alcohol is not particularly limited to a specific one as long as the nitrogen oxide and the alcohol can be mixed together. For example, the nitrogen oxide (particularly the liquid nitrogen oxide) may be added to the alcohol continuously, intermittently, or all at once, or the alcohol may be added to the nitrogen oxide (for example, the liquid nitrogen oxide) continuously, intermittently, or all at once. Moreover, in a reaction of the nitrogen oxide in a gaseous state and the alcohol in a liquid state, the gaseous nitrogen oxide may be bubbled through the liquid alcohol for contacting with each other.

The reaction may be conducted without solvent (in the absence of any solvent) or in a solvent (in the presence of a solvent). An alcohol in a solid state (or an alcohol which solidifies at a reaction temperature) may be directly subjected to the reaction, or the alcohol may be dissolved in a solvent for the reaction. The solvent may usually include a solvent having no active hydrogen group (e.g., a hydroxyl group and/or a carboxyl group), for example, a hydrocarbon-series solvent (e.g., an aliphatic hydrocarbon such as n-hexane or n-heptane; and an aromatic hydrocarbon such as toluene or xylene), an ether-series solvent (e.g., a chain ether such as diethyl ether, and a cyclic ether such as tetrahydrofuran), an ester-series solvent (such as ethyl acetate or methyl acetate), a ketone (e.g., an alkanone such as acetone, methyl ethyl ketone, or diisopropyl ketone), a halogen-containing solvent (e.g., a halogenated hydrocarbon such as methylene chloride or chloroform), a nitro-series solvent (e.g., nitromethane), and a nitrile-series solvent (e.g., acetonitrile). These solvents may be used singly or in combination.

Moreover, the reaction may be conducted in the presence of a nitrite corresponding to the alcohol (or the reaction product) (more specifically, a nitrite which is the same as the reaction product). For example, the nitrite which is the same as the reaction product may be added to the reaction system before or during the reaction. When such a nitrite is in a state of liquid, the nitrite may be used as a solvent, which is also preferrable for the subsequent purification.

The amount of the nitrite to be used may be, for example, about 1 to 200 parts by weight, preferably about 3 to 100 parts by weight (e.g., about 5 to 80 parts by weight), and more preferably about 10 to 60 parts by weight (e.g., about 15 to 50 parts by weight) relative to 100 parts by weight of the amount of the alcohol to be used.

The reaction temperature is, as described above, not particularly limited to a specific one as long as the temperature is lower than 10°C. The reaction temperature may be, for example, not higher than 8°C (e.g., about -40°C to 8°C), preferably not higher than 5°C (e.g., about -30°C to 5°C), more preferably not higher than 3°C (e.g., about -25°C to 3°C), and particularly not higher than 1°C (e.g., about -20°C to 0°C). According to the present invention, a reaction (or contact) of the nitrogen oxide [for example, the liquid nitrogen oxide (particularly, N₂O₃)] and the alcohol at such a low temperature can produce a nitrite efficiently.

Although it is not understood exactly the reason for that the nitrite can be efficiently produced at such a specific range of the reaction temperature, the present invention probably makes use of the instability and easiness of decomposition of the nitrite (particularly, a nitrous acid ester of a secondary or tertiary alcohol). That is, the inventors of the present invention found that the nitrite is easily decomposed with water at a temerature outside the above-mentioned temperature range (e.g., at a temperature of not lower than 10°C). More specifically, the nitrite (particularly, a nitrous acid ester of a secondary or tertiary alcohol) is easily allowed to react with water as by-products at a temperature outside the above-mentioned temperature range (e.g., at a temperature of not lower than 10°C). As a result, the nitrite is decomposed, and the production efficiency is remarkably deteriorated. Moreover, dinitrogen trioxide, which particularly effectively produces the nitrite, begins to vaporize and decompose at about 4 to 6°C. In these regards, the reaction using dinitrogen trioxide at a temperature of not lower than 10°C particularly tends to deteriorate the production efficiency of the nitrite. As mentioned above, the reason for that the nitrite can be obtained in a significantly high yield in the present invention is considered as follows: even when water is present in the reaction system, the produced nitrite is remarkably stable and hardly decomposed, and dinitrogen trioxide most of which are in a liquid state can be allowed to react with the alcohol.

The reaction may be carried out under an atmospheric pressure, a reduced pressure, or an applied pressure. Incidentally, the reaction may be carried out under a reduced pressure as a vapor phase reaction. Moreover, the reaction may be conducted under any atmosphere such as an oxygen atmosphere or an inactive gas atmosphere (such as a nitrogen atmosphere or an argon atmosphere). Incidentally, as described above, in the present invention, even when water (specifically, water produced by the reaction) is present in the reaction system, the nitrite can be produced efficiently. Therefore, in the present invention, the nitrogen oxide can be allowed to react with the alcohol without removing (or substantially removing) water (water produced by the reaction) from the reaction system.

Further, the reaction may be conducted by a batch system, a semi-batch system, or a continuous system.

Such a reaction produces a nitrite as a reaction product. More specifically, the use of the alcohol represented by the above-mentioned formula R(OH)ₙ produces a nitrite represented by the formula (HO)ₖR(ONO)ₘ, where R has the same meaning as defined above, k denotes an integer of not less than 0, m denotes an integer of not less than 1, and k, m, and n satisfies the equation k+m=n.

Incidentally, after the completion of the reaction, the reaction product may be separated and purified by such a conventional means as filtration, condensation, distillation, extraction, crystallization, recrystallization, column chromatography, or a combination means thereof.

In the present invention, the nitrite can be obtained in a high yield. The yield of the nitrite is, for example, not lower than 75% by mole, preferably not lower than 80% by mole, and more preferably not lower than 85% by mole, based on the nitrogen oxide (particularly, the nitrogen oxide at least containing N₂O₃).

According to the present invention, the reaction using the nitrogen oxide under a specific reaction condition can produce a nitrite efficiently. In particular, according to the present invention, even a nitrous acid ester of a secondary or tertiary alcohol can be produced in a high yield.

In the process of the present invention, a nitrite can be produced efficiently not by using a salt of nitrous acid, but by using a nitrogen oxide. In particular, since the process includes a reaction using a liquid nitrogen oxide at a temperature lower than 10°C, even a nitrous acid ester of a secondary alcohol or tertiary alcohol, which cannot be obtained in a sufficient yield by the conventional manner, can be produced in a high yield. Therefore, the process of the present invention is extremely advantageous for producing a nitrite from an industrial viewpoint.

### EXAMPLES

The following examples are intended to describe this invention in further detail and should by no means be interpreted as defining the scope of the invention.

### (Example 1)

A balloon with 5 L of nitrogen monoxide (NO) was attached to a 200 mL four-neck reactor vessel, and the atmosphere in the system was replaced with NO. Then the reactor vessel was cooled to -5°C, and 10 g (217 mmol) of liquid NO₂ was added thereto by a syringe. The mixture was stirred at -5°C for 30 minutes to produce 16.4 g (216 mmol) of N₂O₃ (blue liquid). A mixture of 40 g (540 mmol) of t-butanol and 10 g (97 mmol) of t-butyl nitrite was added dropwise to the resulting liquid N₂O₃ over 30 minutes. Since the reaction was an exothermic reaction, the temperature of the inside of the reactor vessel was controlled at -5°C to 0°C. After the dropwise addition, the reaction product was analyzed by a gas chromatography, which showed that t-butyl nitrite was produced in a yield of 90% (40.1 g, 389 mmol) based on N₂O₃.

### (Example 2)

A balloon with 5 L of nitrogen monoxide (NO) was attached to a 100 mL four-neck reactor vessel, and the atmosphere in the system was replaced with NO. Then the reactor vessel was cooled to -5°C, and 10 g (217 mmol) of liquid NO₂ was added thereto by a syringe. The mixture was stirred at -5°C for 30 minutes to produce 16.4 g (216 mmol) of N₂O₃ (blue liquid). To another 100 mL four-neck flask in which the atmosphere had been replaced with nitrogen were added 40 g (540 mmol) of t-butanol and 10 g (97 mmol) of t-butyl nitrite, and the mixture was cooled to -5°C. The above-described liquid N₂O₃ was transferred into a gas bag and warmed up to a room temperature for vaporization. The vaporized N₂O₃ was bubbled through the flask over one hour. Since the bubbling generated heat, the reaction temperature was controlled at -5°C to 0°C. Incidentally, the vaporized N₂O₃ was immediately liquefied at -5°C to 0°C. That is, the liquid N₂O₃ underwent the reaction. After the completion of the bubbling, the reaction product was analyzed by a gas chromatography, which showed that t-butyl nitrite was newly produced in a yield of 91% (40.5 g, 393 mmol) based on N₂O₃.

### (Comparative Example 1)

A balloon with 5 L of nitrogen monoxide (NO) was attached to a 100 mL four-neck reactor vessel, and the atmosphere in the system was replaced with NO. Then the reactor vessel was cooled to -5°C, and 10 g (217 mmol) of liquid NO₂ was added thereto by a syringe. The mixture was stirred at -5°C for 30 minutes to produce 16.4 g (216 mmol) of N₂O₃ (blue liquid). To another 100 mL four-neck flask in which the atmosphere had been replaced with nitrogen were added 40 g (540 mmol) of t-butanol and 10 g (97 mmol) of t-butyl nitrite, and the temperature of the mixture was adjusted to 25°C. The above-described liquid N₂O₃ was transferred into a gas bag and warmed up to a room temperature for vaporization. The vaporized N₂O₃ was bubbled through the flask over one hour. Since the bubbling generated heat, the reaction temperature was controlled at 25°C to 30°C. After the completion of the bubbling, the reaction product was analyzed by a gas chromatography, which showed that t-butyl nitrite was newly produced in a yield of 31% (13.8 g, 134 mmol) based on N₂O₃.

### (Example 3)

A balloon with 5 L of nitrogen monoxide (NO) was attached to a 100 mL four-neck reactor vessel, and the atmosphere in the system was replaced with NO. Then the reactor vessel was cooled to -5°C, and 10 g (217 mmol) of liquid NO₂ was added thereto by a syringe. The mixture was stirred at -5°C for 30 minutes to produce 16.4 g (216 mmol) of N₂O₃ (blue liquid). To another 100 mL four-neck flask in which the atmosphere had been replaced with nitrogen were added isopropanol (32.5 g, 540 mmol), and the mixture was cooled to -5°C. The above-described liquid N₂O₃ was transferred into a gas bag and warmed up to a room temperature for vaporization. The vaporized N₂O₃ was bubbled through the flask over one hour. Since the bubbling generated heat, the reaction temperature was controlled at -5°C to 0°C. Incidentally, the vaporized N₂O₃ was immediately liquefied at -5°C to 0°C. That is, the liquid N₂O₃ underwent the reaction. After the completion of the bubbling, the reaction product was analyzed by a gas chromatography, which showed that isopropyl nitrite was produced in a yield of 93% (35.8 g, 402 mmol) based on N₂O₃.

### (Comparative Example 2)

A balloon with 5 L of nitrogen monoxide (NO) was attached to a 100 mL four-neck reactor vessel, and the atmosphere in the system was replaced with NO. Then the reactor vessel was cooled to -5°C, and 10 g (217 mmol) of liquid NO₂ was added thereto by a syringe. The mixture was stirred at -5°C for 30 minutes to produce 16.4 g (216 mmol) of N₂O₃ (blue liquid). To another 100 mL four-neck flask in which the atmosphere had been replaced with nitrogen were added isopropanol (32.5 g, 540 mmol), and the temperature of the mixture was adjusted to 25°C. The above-described liquid N₂O₃ was transferred into a gas bag and warmed up to a room temperature for vaporization. The vaporized N₂O₃ was bubbled through the flask over one hour. Since the bubbling generated heat, the reaction temperature was controlled at 25°C to 30°C. After the completion of the bubbling, the reaction product was analyzed by a gas chromatography, which showed that isopropyl nitrite was produced in a yield of 52% (20 g, 225 mmol) based on N₂O₃.

### (Example 4)

A balloon with 5 L of nitrogen monoxide (NO) was attached to a 200 mL four-neck reactor vessel, and the atmosphere in the system was replaced with NO. Then the reactor vessel was cooled to -5°C, and 10 g (217 mmol) of liquid NO₂ was added thereto by a syringe. The mixture was stirred at -15°C for 30 minutes to produce 16.4 g (216 mmol) of N₂O₃ (blue liquid). A mixture of 40 g (540 mmol) of t-butanol and 10 g (97 mmol) of t-butyl nitrite was added dropwise to the resulting liquid N₂O₃ over 30 minutes. Since the reaction was an exothermic reaction, the temperature of the inside of the reactor vessel was controlled at -15°C to -10°C. After the dropwise addition, the reaction product was analyzed by a gas chromatography, which showed that t-butyl nitrite was newly produced in a yield of 94% (41.9 g, 406 mmol) based on N₂O₃.

### (Example 5)

A balloon with 5 L of nitrogen monoxide (NO) was attached to a 200 mL four-neck reactor vessel, and the atmosphere in the system was replaced with NO. Then the reactor vessel was cooled to -5°C, and oxygen gas (217 mmol) was added thereto by a syringe. The mixture was stirred at -5°C for 30 minutes to produce 16.4 g (216 mmol) of N₂O₃ (blue liquid). A mixture of 40 g (540 mmol) of t-butanol and 10 g (97 mmol) of t-butyl nitrite was added dropwise to the resulting liquid N₂O₃ over 30 minutes. Since the reaction was an exothermic reaction, the temperature of the inside of the reactor vessel was controlled at -5°C to 0°C. After the dropwise addition, the reaction product was analyzed by a gas chromatography, which showed that t-butyl nitrite was newly produced in a yield of 90% (40.1 g, 389 mmol) based on N₂O₃.

### (Example 6)

A balloon with 5 L of nitrogen monoxide (NO) was attached to a 200 mL four-neck reactor vessel, and the atmosphere in the system was replaced with NO. Then the reactor vessel was cooled to -5°C, and 10 g (217 mmol) of liquid NO₂ was added thereto by a syringe. The mixture was stirred at -5°C for 30 minutes to produce 16.4 g (216 mmol) of N₂O₃ (blue liquid). A mixture of 15.7 g (135 mmol) of 1,4-cyclohexanediol and acetonitrile (100 mL) was added dropwise to the resulting liquid N₂O₃ over 30 minutes. Since the reaction was an exothermic reaction, the temperature of the inside of the reactor vessel was controlled at -5°C to 0°C. After the dropwise addition, the reaction product was analyzed by a gas chromatography, which showed that dinitrite ester of cyclohexanediol and mononitrite ester of cyclohexanediol were produced in yields of 59% (13.9 g, 80 mmol) and 37% (7.26 g, 50 mmol), respectively, based on cyclohexanediol. The yield of nitrites was 97% based on N₂O₃.

### (Comparative Example 3)

A balloon with 5 L of nitrogen monoxide (NO) was attached to a 100 mL four-neck reactor vessel, and the atmosphere in the system was replaced with NO. Then the reactor vessel was cooled to -5°C, and 10 g (217 mmol) of liquid NO₂ was added thereto by a syringe. The mixture was stirred at -5°C for 30 minutes to produce 16.4 g (216 mmol) of N₂O₃ (blue liquid). To another 100 mL four-neck flask
in which the atmosphere had been replaced with nitrogen were added 40 g of t-butanol (540 mmol) and 10 g of t-butyl nitrite (97 mmol), and the temperature of the mixture was adjusted to 25°C. The above-described liquid N₂O₃ was transferred into a gas bag and warmed up to 12°C for vaporization. The vaporized N₂O₃ was bubbled through the flask over one hour. Since the bubbling generated heat, the reaction temperature was controlled at 10°C to 12°C. After the completion of the bubbling, the reaction product was analyzed by a gas chromatography, which showed that t-butyl nitrite was newly produced in a yield of 52% (23.1 g, 224 mmol) based on N₂O₃.

## Claims

1. A process for producing a nitrite, which comprises allowing a nitrogen oxide to react with an alcohol at a reaction temperature lower than 10°C.

2. A process according to claim 1, wherein the nitrogen oxide contains at least N₂O₃.

3. A process according to claim 1 or 2, wherein the alcohol is an aliphatic secondary alcohol or an aliphatic tertiary alcohol.

4. A process according to any one of claims 1 to 3, wherein the reaction is conducted in the presence of a nitrite corresponding to the alcohol.

5. A process according to any one of claims 1 to 4, wherein the reaction temperature is -25°C to 3°C.
